# EUROPEAN PATENT SPECIFICATION

(11) **EP 1 048 608 B1**
(45) Date of publication and mention of the grant of the patent: **03.10.2007**
(21) Application number: 99830254.1
(22) Date of filing: 29.04.1999
(51) Int. Cl.: B67C 7/00, A61L 2/02

(54) **Continuous method and apparatus for the sterilization of bottled drinks**
Vorrichtung und Verfahren zur kontinuierlichen Sterilisation von in Flaschen abgefüllten Getränken
Dispositif et méthode pour la stérilisation en continu de boissons en bouteilles

(43) Date of publication of application: 02.11.2000
(73) Proprietor: Simonazzi S.p.A., 43040 Parma (IT)
(72) Inventor: Zacche, Vanni, 43015 Noceto-Parma (IT); Preti, Fabrizio, 43015 Noceto-Parma (IT); Colato, Luca, 43015 Noceto-Parma (IT)
(74) Representative: Long, Giorgio

(56) References cited:
- WO-A-95/08275
- WO-A-98/48856
- DE-A- 4 126 136
- DE-A- 4 242 311
- DE-A- 19 612 322

## Description

The present invention relates to a continuous method and associated apparatus for the sterilization of drinks contained in bottles of plastics material, in particular PET bottles.

In practice, there are various methods for the sterilization-pasteurization of drinks and associated containers.

A first method provides for the sterilization of drinks by a thermal treatment and for bottling the drinks at elevated temperature in order to exploit their thermal energy as a sterilizing agent for the container. That method can be used with containers that are capable of retaining their physico-chemical characteristics at the filling temperature and is generally used with containers of glass or aluminium (cans), or recrystallized PET.

A second method provides for the sterilization of drinks by a thermal treatment before bottling and for the subsequent filling of a previously sterilized container at ambient temperature, in an aseptic environment. That system presupposes the sterilization of the container with chemico-physical agents and the maintenance of an aseptic environment during the filling and closing stage by the use of suitable sterile chambers (known as "white chambers") having a volume of several cubic metres, which chambers are difficult to manage in an industrial environment.

A third method encloses the container to be filled into a chamber in which steam is introduced for sterilisation purposes.

The spread in recent years of new dietetic products to which vitamins and/or micro-elements important for supplementing the diet have been added has given rise to the need to provide methods of sterilization other than by heat, in order to avoid irreversible processes denaturing the product.

Among the various methods employed - use of pulsed electric fields, magnetic fields, microwaves, etc., - the method based on the use of hyperbaric chambers in which sterilization is achieved by treatment under high hydrostatic pressure has recently assumed particular importance as it can be seen from WO 98/48856. It is known that the application of high pressure induces morphological changes in the cells of micro-organisms, which may lead to the rupture of the cell membrane and to the consequent death of the micro-organisms themselves.

The extent and the early occurrence of the changes induced by the high pressure depend on various factors, including the intensity of the pressure, the application time and the type of compression/decompression cycle used.

The application to the food industry of the method of sterilization by hyperbaric pressure is known. However, that method has the disadvantage of being discontinuous because it provides for the use of large hyperbaric chambers in which a batch of products to be sterilized is inserted. Furthermore, it is characterized by long sterilization cycle times, in part owing to the time required by the apparatus to reach the operating pressure and to return to atmospheric pressure at the end. It will therefore be appreciated that the known method exhibits poor versatility and does not enable the installation to be sufficiently productive.

The problem underlying the present invention is to provide a method and an apparatus for the sterilization of bottled drinks which are capable of overcoming the disadvantages of the prior art, in particular the problems associated with low productivity.

The problem is solved by a method and by an apparatus that is specifically devised to implement the method, in accordance with the appended claims.

Other characteristics and advantages of the method and the apparatus for the sterilization of bottled drinks forming the subject-matter of the present invention will become clear from the description of some preferred embodiments which are given hereinafter by way of nonlimiting example with reference to the following drawings:
Figure 1 is a diagrammatic plan view of an apparatus for bottling and sterilizing drinks in accordance with the present invention;
Figure 2 is a perspective partial diagrammatic view of the sterilization unit forming the subject-matter of the present invention;
Figure 3 is a sectional perspective view of a pressurization device according to the present invention;

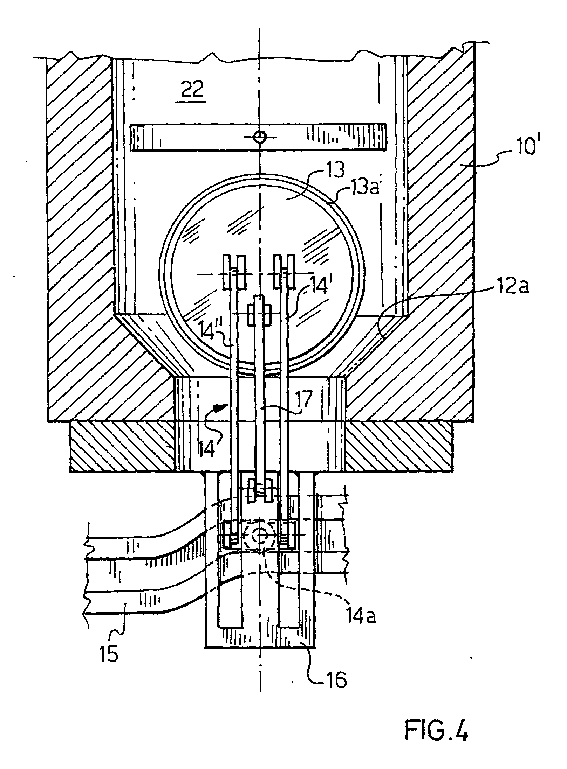

Figure 4 is a sectional side view of the detail of the door of the device of Figure 3;
Figures 5a-5c show the sequence for the introduction of the bottle into the pressurization device of Figure 3, according to a sectional front view thereof;
Figure 6 is the graph of a possible cycle of compressions and decompressions according to the method of the present invention;
Figure 7 is the graph of a second possible cycle of compressions and decompressions according to the method of the present invention.

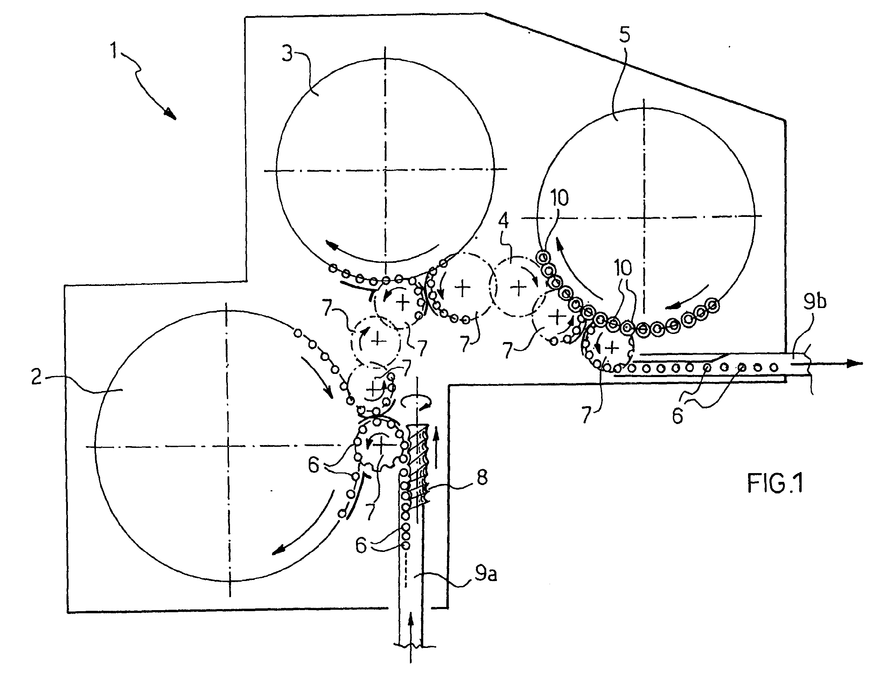

Referring to Figure 1, the apparatus, generally indicated 1, for filling and sterilizing bottles according to the present invention comprises a rinsing unit 2, a filling unit 3, a capsule-fitting device 4 and a sterilization apparatus 5.

The apparatus 1 permits the continuous filling and sterilization of bottles 6 and may be in a rotary form, as shown in Figure 1, or alternatively in a linear form, in which the various operating units are arranged in sequence along a conveyor belt.

The apparatus of Figure 1 also comprises a plurality of star-shaped transfer units 7 which transfer the individual bottles to and from the operating units 2, 3, 4 and 5, in accordance with methods that are well known and used in machines of this type.

A screw spacer 8 is arranged in the region of the first of those transfer units 7, which transports incoming bottles to the rinsing unit 2. The function of the screw spacer 8 is to change the pitch of arrival of the bottles 6 from a state of substantial contact to a separated state required by the subsequent processing in the operating units 2, 3, 4 and 5.

The bottles 6 are fed to the apparatus 1 by means of a transport system 9a and, when processing is complete, they are removed by means of a second transport system 9b. The transport systems 9a, 9b are of known type and will therefore not be described in any further detail. Analogously, the rinsing 2, filling 3 and capsule-fitting 4 units, like the transfer units 7, are of known type and therefore do not require more detailed description, since they do not individually constitute subject-matter of the present invention.

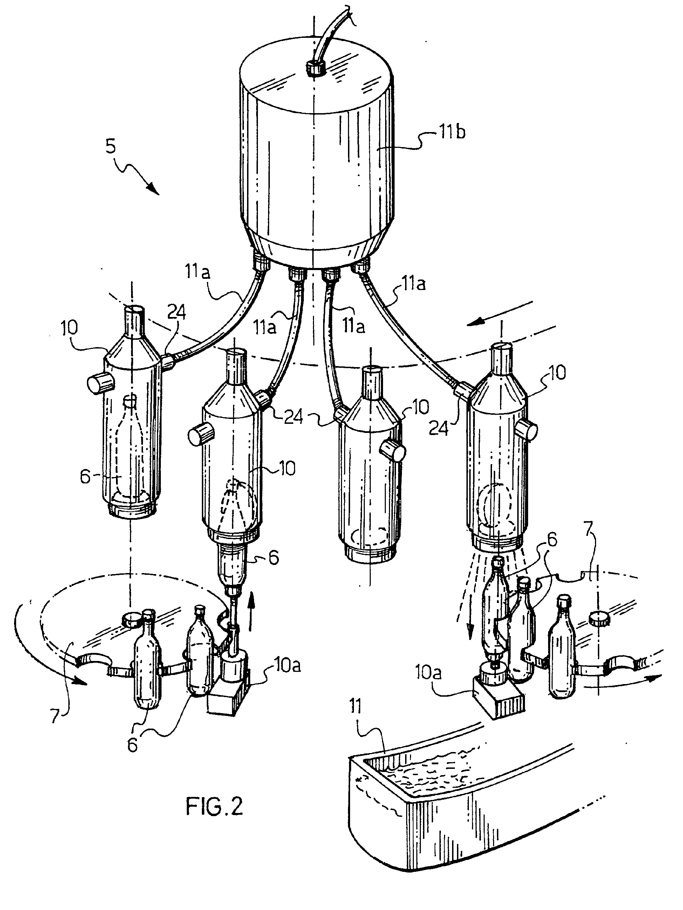

With reference to Figures 1 and 2, the apparatus for sterilization 5 comprises a plurality of pressurization devices 10 arranged at a mutual interaxial distance corresponding to the interaxial spacing at which the bottles 6 are supplied. The pressurization devices 10 are secured in known manner to the rotating structure of the sterilization unit 5 (not shown in the drawing). The rotating structure (or turntable) is of known type and is widely used in rotary machines. A jack 10a operated by a respective cam (not shown in the drawing) is arranged below each pressurization device 10 and in alignment with the longitudinal axis thereof.

In the lower portion of the sterilization apparatus 5, below the sterilization devices 10 and the jacks 10a, is a channel 11 for collecting water which is connected by means of a pump (not shown in the drawing) to a water tank 11b which is preferably arranged above the sterilization devices 10 in a central position relative to the sterilization apparatus 5. The water tank 11b is connected by means of a plurality of tubes 11a to the individual devices 10 by means of valves 24, as will be described more clearly hereinafter.

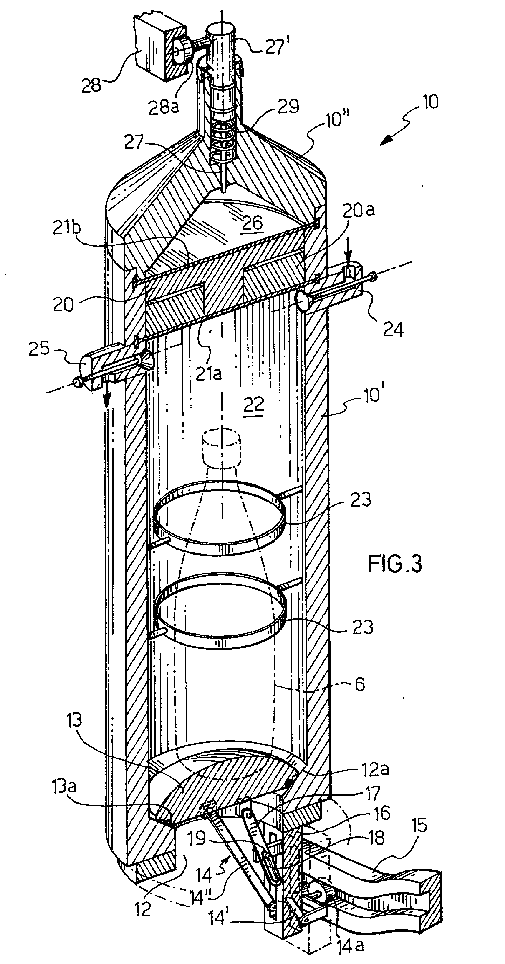

Referring now to Figure 3, each individual sterilization device 10 comprises a substantially cylindrical hollow body 10' having a lower opening 12. The opening 12 has an internal edge which is flared to form a conical seat 12a. The top of the body 10' comprises a closure cover 10" which is preferably frustoconical.

The device 10 is produced from a material having high mechanical strength, such as, for example, stainless steel of a thickness sufficient to withstand the high pressures generated inside.

The opening 12 is closed by a door 13 having a preferably hemispherical convex upper surface. The lower surface of the door 13 has flared edges complementing the conical seat 12a in order to obtain a closure of the sterilization device 10 capable of ensuring sealing even during the internal pressurization stage. An annular seat 13a that is to accommodate a suitable seal, for example a rubber O-ring, is also formed on the surface of the flared edges. The seal ensures leakproofness even during the stage of filling the device 10 with water under atmospheric pressure.

Hinged to the lower surface of the door 13 is an operating arm 14 which is in turn connected to a roller cam 15 (a detail of which is shown in Figure 2) by means of a suitable driven member 14a terminating in a small wheel. The driven member 14a is also slidably engaged on a guide support 16, in turn secured to the hollow body 10', which permits the guided translation thereof in the vertical direction.

As shown in Figure 3, the operating arm 14 is divided into two parallel arms 14' and 14" for reasons which will emerge more clearly hereinafter.

A link 17 provided with a slot 18 is also hinged to the lower surface of the door 13. A pin 19 secured to the guide support 16 is inserted in the slot 18. The link 17 is arranged in an intermediate position relative to the arms 14' and 14" in order to permit the crossing of the operating arm 14 and the link 17 during the kinematic opening motion of the door 13.

A piston 20 having a T-shaped cross-section is slidably inserted in the upper portion of the hollow body 10'. Resilient walls 21a and 21b having a sealing function are arranged below and above the piston 20 and in contact therewith. The piston 20 is also held vertically by the lower wall 21a. The resilient walls 21a, 21b may, for example, be produced from a suitable oil-resistant plastics material.

An annular shoulder 20a which protrudes from the internal walls of the hollow body 10' is arranged below the portion of larger diameter of the piston 20. The annular shoulder 20a has the function of limiting the stroke of the piston during the pressurization stage. It will therefore be appreciated that, in the rest state, there will be a gap between the annular shoulder 20a and the lower surface of the portion of larger diameter of the piston 20.

A pressurization chamber 22 suitable for accommodating a bottle 6 is defined inside the hollow body 10', below the wall 21a. Centring means 23 for the bottle, such as, for example, the rings shown in Figure 2 or, alternatively, a stand, which are secured to the internal wall of the chamber, are provided inside the pressurization chamber 22. The function of the centring means 23 is to keep the bottle 6 upright and aligned with the longitudinal axis of the device 10 during the various treatment stages.

A valve 24 having a mushroom-shaped closure element is accommodated on the wall of the upper portion of the pressurization chamber 22, just below the wall 21a. The function of the valve 24 is to regulate the flow of water which is admitted into the chamber 22 before the pressurization stage. The valve 24 is therefore connected to the tank 11b by way of the tube 11a. Alternatively, the valve 24 can be connected directly to the water mains.

A second valve 25 having a mushroom-shaped closure element is arranged in the same upper portion of the pressurization chamber 22 but preferably spaced from the first valve 24. The function of the valve 25 is to permit the discharge of residual air during the stage of filling with water.

The choice of the mushroom-shaped closure element and the associated conical seat enables a high degree of sealing of the valves to be achieved even during the stage of operation at high pressure.

A chamber 26 which is to be completely filled with an incompressible liquid is defined inside the hollow body 10', above the wall 21b.

Formed in the closure cover 10" is a seat for a piston 27 which is operated by a roller cam 28 (only a detail of which is shown in Figure 2) by means of a driven member 28a terminating in a small wheel.

The piston 27 has an upper portion 27' of larger diameter which is in turn accommodated in an associated seat. A shoulder, on which a spring dynamometer 29 bears, is thus formed in the region of the junction between the portion of larger diameter 27' and the portion of smaller diameter of the piston 27.

It is vital that the piston 27 has a cross-section having a far smaller surface area than that of the wall 21b, for reasons which will emerge hereinafter.

Still referring to the drawings, the functioning of the apparatus for sterilizing bottled drinks, which forms the subject-matter of the present invention, is as follows.

The sterilization apparatus 5 is constituted by a turntable which rotates in the direction shown in Figure 2 and on which the sterilization devices 10 are secured.

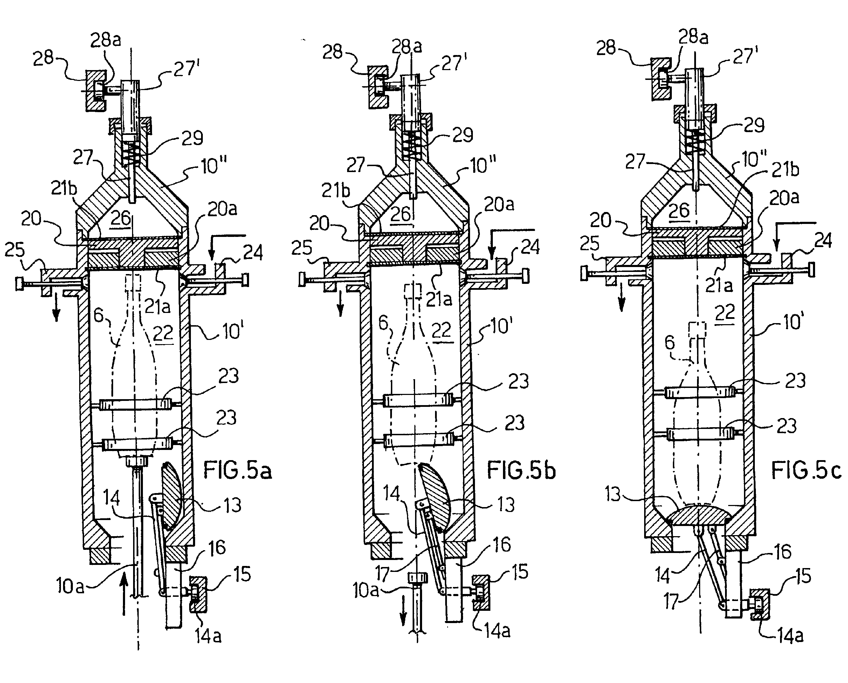

The bottle 6 arriving at the sterilization unit 5 is transferred by the star-shaped transfer unit 7 to the pressurization device 10 by means of the jack 10a (as shown in Figures 2 and 5a). The device 10 is empty in that position and the door 13 is open.

At that point, as shown in Figure 5b, the door 13 starts to close owing to the action of the operating arm 14 moved by the cam 15. At the same time, the jack 10a starts to move back in order to enable the bottle 6 to be engaged by the convex surface of the door 13. In order to perform that movement, the cam 15 provides for a first negative lift which, as stated, enables the door 13 to be placed below the base of the bottle in the position shown in Figure 5b, thus a stationary stage, in which the door 13 remains at a standstill. In the stationary stage, as stated, the jack moves back and the base of the bottle engages with the convex surface of the door 13. The cam then performs a second negative lift which enables the door 13 to close completely. The convex surface of the door 13 enables continuous contact to be maintained with the base of the bottle, accompanying the bottle as far as the closure position of the door, as shown in Figure 5c.
The centring means 23 keep the bottle 6 permanently upright and aligned with the longitudinal axis of the hollow body 10'.

The kinematic opening and closing motion of the door 13 is known per se and is obtained by means of the combined action of the operating arm 14 and the link 17. In practice, the double hinging of the door, to the operating arm 14 and to the link 17, enables it to assume a position which is upright and rotated when the door is open and a position which is horizontal and aligned with the opening 12 when the door is closed. Furthermore, the presence of the slot 18 in sliding engagement with the pin 19 enables the door to perform a rectilinear vertical movement in the final stages of closing the door (or in the initial stage of opening it) so that the lower surface of the door 13 engages correctly with the conical seat 12a. In that stage, the pin 19 slides along the slot 18 and therefore does not exert any force on the door. However, once the pin 19 has reached the end of the slot 18, it acts as a hinging point for the door 13 and causes it to be rotated about the axis passing through the pin.

Returning to the functioning of the device 10, once the stage of closing the door 13 has been completed, the valve 24 through which water is admitted to the inside of the pressurization chamber 22 is opened. At the same time, the valve 25 for discharging the residual air from the inside of the pressurization chamber 22 is also opened. Once the filling of the pressurization chamber has been completed, this being detectable by the escape of water from the valve 25, the valves 25 and 24 are closed in sequence. The seal accommodated in the seat 13a of the door prevents water escaping from the bottom during the charging stage which takes place at atmospheric pressure.

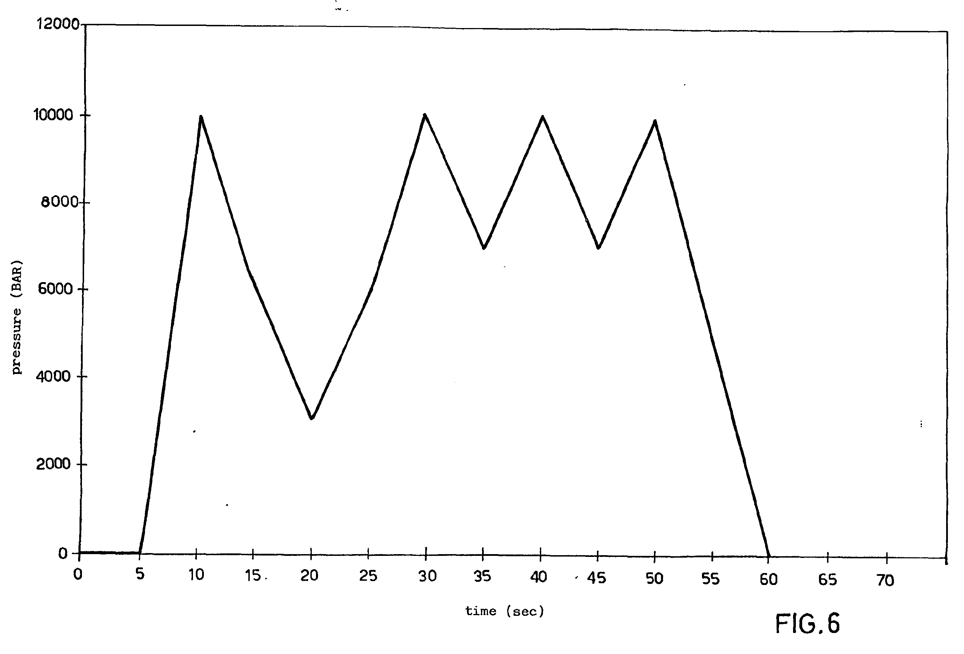

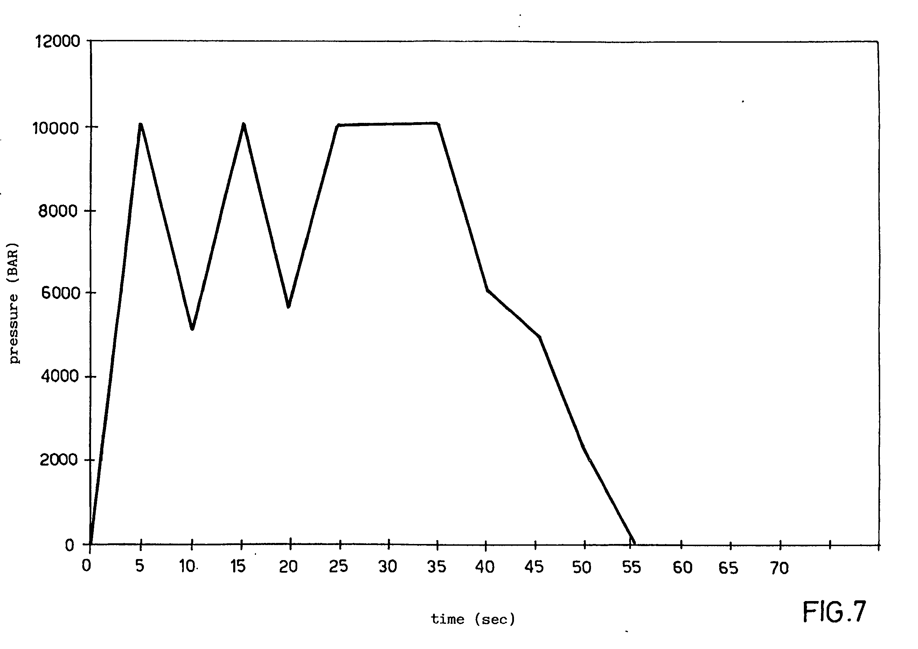

At that point, the pressurization stage starts. By means of the cam 28, the piston 27 performs a predetermined stroke inside the chamber 26, which has previously been filled with an incompressible fluid. Thus, the pressure imparted is amplified on the resilient wall 21b in accordance with Pascal's principle. A second amplification is obtained by the action of the portion of smaller diameter of the piston 20 on the wall 21a, the latter being in direct contact with the fluid in the pressurization chamber 22. In that manner, pressures as high as 10,000 bar are obtained inside the pressurization chamber 22. The decompression stage is obtained simply by returning the piston 27 into its seat. The operation can be repeated, carrying out a cycle of compressions and decompressions, preferably not lasting more than 60 seconds, which kills the micro-organisms present in the drink in an extremely short space of time. Figures 6 and 7 show two examples of compression-decompression cycles which can be carried out for the purposes of the present invention. As will be appreciated, the points of maximum pressure (10,000 bar) are followed by stages of partial decompression. The pressure is returned to atmospheric pressure only at the end of the cycle.

In order to reduce the times necessary for sterilization, the pressurization chamber 22 may be filled with water preheated to approximately 40°C. For that purpose, the tank 11b may be provided with suitable heating means. Analogously, the drink may be bottled beforehand at that temperature.

A specific initial pressure imparted by the piston 27, which pressure is in turn dictated by the stroke of the piston inside the chamber 26, corresponds to a predetermined pressure inside the pressurization chamber 22. It is, for example, advantageous for the initial pressure imparted by the piston 27 to be 500 kg/cm². However, it is necessary for the piston stroke to be set in such a manner as to produce a higher pressure, because, owing to the fact that the bottles 6 are never completely filled, the pressure created inside the pressurization chamber 22 may in part be absorbed by the compression of the residual gas inside the bottle. However, in order to prevent overpressures, caused by the fact that the degree of filling is generally different from bottle to bottle, and that it is consequently impossible to provide for an initial pressure of the piston 27 which corresponds to one specific pressure inside the pressurization chamber 22, the spring dynamometer 29 has been provided. The dynamometer 29, set at a pressure, for example, of 500 kg/cm², ensures a homogeneous pressure inside the pressurization chamber 22 by absorbing any internal overpressures.

When the cycle of compressions and decompressions has been completed, the bottle 6 is removed from the device 10, which is at that point in the vicinity of the star-shaped transfer unit 7 which removes the bottles from the sterilization apparatus 5. The operations already described for charging the bottle are repeated in reverse order (see Figures 5a-5c). When the door 13 is opened, the water present inside the pressurization chamber 22 is collected by the channel 11 and recirculated to the tank 11b.

A control and monitoring unit (not shown in the drawings) supervises the functioning of the entire apparatus and correctly effects the sequence of operations. The control and monitoring unit is of known type and therefore will not be described in more detail.

It will be appreciated that the embodiment that has been described is only one particular embodiment of the apparatus for sterilizing bottled drinks which forms the subject-matter of the present invention and to which the expert in the art can introduce any necessary modifications to adapt it to particular applications without, moreover, departing from the scope of protection of the present invention.

For example, it would be possible to replace the spring dynamometer 29 by a dynamometric device of a different type, such as a dynamometer using air, previously compressed to a predetermined pressure.

The removal of the bottle from the device 10 could take place simply by gravity, collecting it in a bag suitably arranged beneath it.

## Claims

1. An apparatus for the continuous sterilization of bottled drinks, of the rotary or linear type, comprising a filling unit (3) for filling the bottles with drinks, a capsule-fitting device (4) for closing the bottles, and a plurality of sterilization devices (10) arranged along the operating line of the apparatus, each of the sterilization devices (10) being fashioned to contain a bottle (6) to be sterilized **characterized in that** the filling unit 3 and the capsule-fitting device 4 are placed upstream with respect to the sterilization devices (10).

2. An apparatus according to claim 1, wherein the sterilization device (10) is a device for the generation of high hydrostatic pressure.

3. An apparatus according to claim 2, wherein the sterilization device (10) comprises a hollow body (10') which defines in its interior a pressurization chamber (22), the pressurization chamber (22) being closed at the bottom by a door (13), valve means (24, 25) being provided for the inlet of water and the escape of air, and the sterilization device (10) comprising means for the generation of high hydrostatic pressure.

4. An apparatus according to claim 3, wherein the means for the generation of high hydrostatic pressure comprise a device for amplifying hydrostatic pressure.

5. An apparatus according to claim 4, wherein the device for amplifying hydrostatic pressure comprises a first piston (27) connected to a cam (28), a chamber (26) filled with an incompressible fluid in which the first piston (27) acts, the chamber (26) being delimited at the bottom by a resilient wall (21b), below and in contact with which is arranged a second piston (20), the second piston (20) being supported at the bottom by a second resilient wall (21a) which separates it from the pressurization chamber (22), the first piston (27) having a cross-section having a smaller surface area than the surface area of the resilient wall (21b).

6. An apparatus according to claim 5, wherein the second piston (20) has a T-shaped cross-section.

7. An apparatus according to claim 5 or 6, wherein the first piston (27) has an upper portion (27') of larger diameter on which a spring dynamometer (29) bears.

8. An apparatus according to any one of claims 1 to 7, wherein centring means (23) for the bottle (6) are secured inside the sterilization device (10) , the centring means (23) being fashioned in such a manner as to keep the bottle (6) upright and aligned with the longitudinal axis of the device.

9. An apparatus according to any one of claims 3 to 8, wherein the door (13) has a convex upper surface which is preferably hemispherical and which opens inwardly.

10. An apparatus according to any one of claims 3 to 9, wherein the door (13) is hinged at the bottom to an operating arm (14) and to a link (17), the operating arm (14) being connected to a cam (15).

11. An apparatus according to any one of claims 1 to 10, wherein each of the sterilization devices (10) is connected to a water tank (11b) by means of a tube (11a).

12. An apparatus according to any one of claims 1 to 11, wherein a jack (10a) is arranged below each sterilization device (10), in alignment with the longitudinal axis of the device.

13. An apparatus according to any one of claims 1 to 12, wherein a channel (11) for collecting the water discharged from the device at the end of the operation is arranged below the sterilization devices (10).

14. A sterilization device (10) as described in any one of claims 3 to 10.

15. A method for the continuous sterilization of bottled drinks, comprising the stages of:
- providing an apparatus according to claim 1 or 2;
- introducing a bottle (6) into each sterilization device (10);
- performing a cycle of compressions and decompressions inside the sterilization device (10);
- removing the bottles (6) from each of the sterilization devices (10)
wherein the bottle is filled with the drink and closed before said step of introducing a bottle (6) into each sterilization device (10).

16. A method according to claim 15, wherein the cycle of compressions and decompressions does not last more than 60 seconds and wherein the pressure is up to 10,000 bar.

## Patentansprüche

1. Vorrichtung zur fortlaufenden Sterilisation von in Flaschen abgefüllten Getränken in rotierender oder linearer Ausführung, aufweisend eine Befülleinheit (3) zum Befüllen der Flaschen mit Getränken, eine Einrichtung (4) zum Anbringen des Flaschenverschlusses zum Verschließen der Flaschen und eine Vielzahl von Sterilisationsgeräten (10), die entlang des Verarbeitungsbands der Vorrichtung angeordnet sind, wobei jedes der Sterilisationsgeräte (10) so gestaltet ist, dass es eine zu sterilisierende Flasche (6) enthält, **dadurch gekennzeichnet, dass** die Befüllungseinheit (3) und die Einrichtung (4) zum Anbringen des Flaschenverschlusses den Sterilisationsgeräten (10) vorgelagert angeordnet sind.

2. Vorrichtung gemäß Anspruch 1, wobei das Sterilisationsgerät (10) eine Vorrichtung zum Erzeugen eines hohen hydrostatischen Drucks ist.

3. Vorrichtung gemäß Anspruch 2, wobei das Sterilisationsgerät (10) einen Hohlkörper (10'), der im Inneren eine Druckkammer (22) definiert, wobei die Druckkammer (22) an der Unterseite durch eine Tür (13) verschlossen wird, und Ventilmittel (24, 25), die für den Wassereinlauf und die Entlüftung ausgebildet sind, umfasst und das Sterilisationsgerät (10) Mittel zum Erzeugen hohen hydrostatischen Drucks umfasst.

4. Vorrichtung gemäß Anspruch 3, wobei die Mittel zum Erzeugen von hohen hydrostatischem Druck eine Einrichtung zum Erhöhen des hydrostatischen Drucks umfassen.

5. Vorrichtung gemäß Anspruch 4, wobei die Einrichtung zum Erhöhen des hydrostatischen Drucks einen ersten Kolben (27) umfasst, der mit einem Nocken (28) verbunden ist, eine Kammer (26), die mit einer inkompressiblen Flüssigkeit gefüllt ist, in der sich der erste Kolben (27) bewegt, wobei die Kammer (26) an der Unterseite durch eine elastische Wand (21b) begrenzt ist, unterhalb derer und im Kontakt mit der ein zweiter Kolben (20) angeordnet ist, wobei der zweite Kolben (20) an der Unterseite durch eine zweite elastische Wand (21a) gehalten wird, die ihn von der Druckkammer (22) trennt, wobei der erste Kolben (27) einen Querschnitt mit einer kleineren Fläche wie die Fläche der elastischen Wand (21b) aufweist.

6. Vorrichtung gemäß Anspruch 5, wobei der zweite Kolben (20) einen T-förmigen Querschnitt aufweist.

7. Vorrichtung gemäß Anspruch 5 oder 6, wobei der erste Kolben (27) einen oberen Bereich (27') mit einem breiteren Durchmesser aufweist, an dem ein Federdynamometer (29) befestigt ist.

8. Vorrichtung gemäß einem der Ansprüche 1 bis 7, wobei Zentriermittel (23) für die Flasche (6) innen im Sterilisationsgerät (10) befestigt sind, wobei die Zentriermittel (23) dergestalt ausgebildet sind, dass die Flasche (6) aufrecht steht und an der Längsachse der Vorrichtung ausgerichtet bleibt.

9. Vorrichtung gemäß einem der Ansprüche 3 bis 8, wobei die Tür (13) eine konvexe Oberseite aufweist, die vorzugsweise halbkugelförmig ist, und die Tür sich nach innen öffnet.

10. Vorrichtung gemäß einem der Ansprüche 3 bis 9, wobei die Tür (13) an der Unterseite gelenkig mit einem Betriebsarm (14) und einem Verbindungsstück (17) verbunden ist, wobei der Betriebsarm (14) mit einem Nocken (15) verbunden ist.

11. Vorrichtung gemäß einem der Ansprüche 1 bis 10, wobei jedes Sterilisationsgerät (10) durch eine Rohrleitung (11a) mit einem Wassertank (11b) verbunden ist.

12. Vorrichtung gemäß einem der Ansprüche 1 bis 11, wobei unterhalb eines jeden Sterilisationsgeräts (10) ein Heber (10a) in Ausrichtung mit der Längsachse des Geräts angeordnet ist.

13. Vorrichtung gemäß einem der Ansprüche 1 bis 12, wobei ein Kanal (11) zur Aufnahme des aus dem Gerät am Ende des Arbeitsgangs abgelassenen Wassers unterhalb der Sterilisationsgeräte (10) angeordnet ist.

14. Sterilisationsgerät (10) wie in einem der Ansprüche 3 bis 10 beschrieben.

15. Verfahren zur fortlaufenden Sterilisation von in Flaschen abgefüllten Getränken, die Stufen umfassend:
- Ausbilden einer Vorrichtung gemäß Anspruch 1 oder 2;
- Einfügen einer Flasche (6) in jedes Sterilisationsgerät (10);
- Durchführen eines Zyklus aus Druckaufbau- und Druckabbauvorgängen im Sterilisationsgerät (10);
- Entfernen der Flaschen (6) aus jedem der Sterilisationsgeräte (10), wobei die Flasche mit einem Getränk befüllt und geschlossen ist, bevor der Schritt ausgeführt wird, in jedes Sterilisationsgerät (10) eine Flasche (6) einzufügen.

16. Verfahren gemäß Anspruch 15, wobei der Zyklus aus Druckaufbau- und Druckabbauvorgängen nicht länger als 60 Sekunden dauert und der Druck bis zu 10.000 bar hoch ist.

## Revendications

1. Dispositif de stérilisation en continu de boissons embouteillées, du type rotatif ou linéaire, comprenant une unité de remplissage (3) destinée à remplir les bouteilles avec des boissons, un dispositif d'adaptation de capsules (4) destiné à fermer les bouteilles et une pluralité de dispositifs de stérilisation (10) agencés le long de la chaîne de production du dispositif, chacun des dispositifs de stérilisation (10) étant conçu pour contenir une bouteille (6) devant être stérilisée, **caractérisé en ce que** l'unité de remplissage (3) et le dispositif d'adaptation de capsules (4) sont placés en amont par rapport aux dispositifs de stérilisation (10).

2. Dispositif selon la revendication 1, dans lequel le dispositif de stérilisation (10) est un dispositif destiné à la génération d'une pression hydrostatique élevée.

3. Dispositif selon la revendication 2, dans lequel le dispositif de stérilisation (10) comprend un corps creux (10') qui définit dans son intérieur une chambre de mise sous pression (22), la chambre de mise sous pression (22) étant fermée au fond par une trappe (13), un moyen de vanne (24, 25) étant prévu pour l'entrée d'eau et la sortie d'air, et le dispositif de stérilisation (10) comprenant un moyen destiné à la génération d'une pression hydrostatique élevée.

4. Dispositif selon la revendication 3, dans lequel le moyen destiné à la génération d'une pression hydrostatique élevée comprend un dispositif destiné à amplifier la pression hydrostatique.

5. Dispositif selon la revendication 4, dans lequel le dispositif destiné à amplifier la pression hydrostatique comprend un premier piston (27) relié à une came (28), une chambre (26) remplie d'un fluide incompressible dans lequel le premier piston (27) agit, la chambre (26) étant délimitée au fond par une paroi élastique (21b), en dessous de laquelle et en contact avec laquelle est agencé un second piston (20), le second piston (20) étant supporté au fond par une seconde paroi élastique (21a) qui le sépare de la chambre de mise sous pression (22), le premier piston (27) présentant une section transversale présentant une aire de surface plus petite que l'aire de surface de la paroi élastique (21b).

6. Dispositif selon la revendication 5, dans lequel le second piston (20) présente une section transversale en forme de T.

7. Dispositif selon la revendication 5 ou 6, dans lequel le premier piston (27) comporte une partie supérieure (27') d'un diamètre plus important sur laquelle un dynamomètre à ressort (29) repose.

8. Dispositif selon l'une quelconque des revendications 1 à 7, dans lequel un moyen de centrage (23) de la bouteille (6) est fixé à l'intérieur du dispositif de stérilisation (10), le moyen de centrage (23) étant conçu de manière à maintenir la bouteille (6) verticale et alignée avec l'axe longitudinal du dispositif.

9. Dispositif selon l'une quelconque des revendications 3 à 8, dans lequel la trappe (13) présente une surface supérieure convexe qui est de préférence hémisphérique et qui s'ouvre vers l'intérieur.

10. Dispositif selon l'une quelconque des revendications 3 à 9, dans lequel la trappe (13) est reliée par articulation au niveau du fond à un bras de commande (14) et à un élément de liaison (17), le bras de commande (14) étant relié à une came (15).

11. Dispositif selon l'une quelconque des revendications 1 à 10, dans lequel chacun des dispositifs de stérilisation (10) est relié à un réservoir d'eau (11b) au moyen d'un tube (11a).

12. Dispositif selon l'une quelconque des revendications 1 à 11, dans lequel un vérin (10a) est agencé en dessous de chaque dispositif de stérilisation (10), en alignement avec l'axe longitudinal du dispositif.

13. Dispositif selon l'une quelconque des revendications 1 à 12, dans lequel un canal (11) destiné à récupérer l'eau évacuée du dispositif à la fin de l'opération est agencé au-dessous des dispositifs de stérilisation (10).

14. Dispositif de stérilisation (10) selon l'une quelconque des revendications 3 à 10.

15. Procédé de stérilisation en continu de boissons embouteillées, comprenant les étapes consistant à :
- fournir un dispositif selon la revendication 1 ou 2 ;
- introduire une bouteille (6) dans chaque dispositif de stérilisation (10) ;
- exécuter un cycle de compressions et de décompressions à l'intérieur du dispositif de stérilisation (10) ;
- retirer les bouteilles (6) de chacun des dispositifs de stérilisation (10),
dans lequel la bouteille est remplie avec la boisson et fermée avant ladite étape d'introduction d'une bouteille (6) dans chaque dispositif de stérilisation (10).

16. Procédé selon la revendication 15, dans lequel le cycle de compressions et de décompressions ne dure pas plus de 60 secondes et dans lequel la pression monte jusqu'à 10 000 bars.
